# EUROPEAN PATENT APPLICATION

(11) **EP 3 922 702 A1**
(43) Date of publication of application: **15.12.2021**
(21) Application number: 20179614.1
(22) Date of filing: 12.06.2020
(51) Int. Cl.: C11B 11/00, A61K 36/73, A23K 10/30, A23K 20/10

(54) **PROCESS FOR OBTAINING EXTRACTS OF CONDENSED TANNINS FROM AERIAL PART OF CISTUS LADANIFER L**

(30) Priority: 08.06.2020 PT 2020116479
(71) Applicant: Cebal - Centro De Biotecnologia Agrícola E Agro-Alimentar Do Alentejo, 7801-908 Beja (PT)
(72) Inventor: ROSA FRAGOSO DAS NEVES GUERREIRO, OLINDA, 7800-344 BEJA (PT); PEREIRA DUARTE RICARDO, MARIA DE FÁTIMA, 7800-073 BEJA (PT); SOUSA JERÓNIMO ALVES, ELIANA ALEXANDRA, 7670-354 OURIQUE (PT)
(74) Representative: Ferreira, Maria Silvina

(57) **Abstract**

The present application intends relates to method for obtaining extracts of condensed tannins from aerial part of *Cistus ladanifer.* The invention contemplates the extraction of condensed tannins, present in aerial part of *Cistus ladanifer,* where, before the extractive process, the biomass is dried and ground to a particle size less than 1 mm (1). After this preparation, the biomass undergoes the extraction procedure, with an ultrasonic probe (2, 4) and using a mixture of acetone:water as extraction solvent. The extraction process is controlled in relation to time, temperature and solid:liquid ratio. This process for obtaining extracts of condensed tannins from *Cistus ladanifer* have potential for application in the feedstuffs industry.

## Description

### Technical field

This application relates to a process to obtain extracts containing condensed tannins and the relating process of extraction involving the use of aerial part of *Cistus ladanifer* L. and the ultrasound-assisted method.

### Background art

Condensed tannins, also known as proanthocyanidins, are complexes of oligomers and polymers composed of flavans-3-ols and flavan-3,4-diols, linked by carbon-carbon bonds. Condensed tannins molecular weight ranges from 1000 to 20000 Da (Hagerman, 1992).

The use of condensed tannins in feedstuffs are of concern, because tannins are generally considered as antinutritive and/or toxic compounds. However, depending on several factors as condensed tannins chemical structure and concentration in diets, composition of basal diet, and factors related to the animal, the condensed tannins might have beneficial effects in ruminants, such as improve the digestive utilization of feed proteins, enhance the growth performance, wool growth, and milk production, improve the animal antioxidant status and oxidative stability of products due to its antioxidant properties and increase the health fatty acid levels in ruminant fat (Jerónimo *et al.,* 2016; Min, Barry, Attwood, & McNabb, 2003; Vasta & Luciano, 2011) .

*Cistus ladanifer* L., also known as rockrose, is a perennial shrub quite abundant in Mediterranean region, that contain high levels of phenolic compounds, as condensed tannins (40 to 160 g/kg dry matter). Some *in vitro* and *in vivo* studies were performed to explore the application of *Cistus ladanifer* condensed tannins extract in ruminant diets, particularly as way to enhance the digestive utilization of dietary protein and to improve fatty acids profile of the ruminant products (Dentinho, Belo, & Bessa, 2014; Dentinho *et al.,* 2020; Guerreiro *et al.,* 2016; Guerreiro *et al.,* 2020).

There are available several methods for extraction of phenolic compounds as condensed tannins. However, extraction of condensed tannins from *Cistus ladanifer* only was reported in four studies (Dentinho *et al.,* 2014; Dentinho *et al.,* 2020; Guerreiro *et al.,* 2016; Guerreiro *et al.,* 2020). The processes described on these works are time consuming, and two of them (Dentinho *et al.,* 2014; Guerreiro *et al.,* 2016) require a combination of multiple extractions steps, using several types of organic solvents, associated by one step wherein a solution containing the condensed tannins is absorbed on a macroreticular polymeric resin, where the absorbed liquid is subsequently eluted and concentrated.

In 2014, the first extraction of condensed tannins from *Cistus ladanifer* was published, where the extraction from aerial part was carried out using solid:liquid batch (1/8, solid:liquid ratio) extraction, with aqueous acetone solution (70:30, acetone:water) as solvent and a extraction time of 3 h. After acetone removal, the aqueous extract was washed with petroleum ether to remove fats and pigments, followed by the evaporation of the remaining organic solvent. Then, absolute ethanol was added to the aqueous extract and this mixture was applied into a Sephadex LH-20 chromatographic column and it is eluted, first, with aqueous ethanol solution (95:5, ethanol:water) and, second, with aqueous acetone solution (70:30, acetone:water). Finally, acetone was removed by evaporation and the aqueous extract was freeze-dried, obtaining the condensed tannins extract (Dentinho *et al.,* 2014). The proposed invention differs from this work for not use additional extraction steps neither use of environmentally aggressive extraction solvents, obtaining an extract of condensed tannins only with ultrasound extraction and using only the aqueous acetone solution as extraction solvent.

Later in 2016, aerial part of *Cistus ladanifer* were submitted to a sequential extraction (Guerreiro *et al.,* 2016). Where, the aerial part was first submitted to steam distillation for 6 h, for extraction of the essential oils, and the solid residue obtained was freeze-dried and ground (1 mm). Then, the solid residue was extracted with dichloromethane in a Soxhlet extractor for 7 h. Afterwards, the leftover residue was extracted using a solid:liquid batch (1/5, solid:liquid ratio) extraction, with aqueous acetone solution (70:30, acetone:water), at room temperature, for 8 h. The extract was then centrifuged and the leftover residue was washed with aqueous acetone solution and centrifuged. The supernatants were pooled and filtered by vacuum. The fraction composed by all extracted phenolic compounds (called as total phenolic fraction) was obtained by sequential removal of acetone, by rotary evaporation, and the water by freeze-drying. The total phenolic fraction was then applied to a Sephadex LH-20 chromatographic column, which was eluted with a aqueous ethanol solution (95:5, ethanol:water), and finally, it was eluted with aqueous acetone solution (70:30, acetone:water). The solvent was evaporated by rotary evaporation and the water was removed by freeze-drying, obtaining the condensed tannins fraction. The present invention is distinct from this study for obtaining an extract of condensed tannins without use of environmentally aggressive extraction solvents, and without use of additional extraction steps, which become the process described by Guerreiro et al. (2016) in a time consuming and expensive process.

More recently, aerial part of *Cistus ladanifer* were dried and ground (1 mm). Then, the extraction from aerial part was carried out using a solid:liquid batch (1/5, solid:liquid ratio) extraction, with aqueous acetone solution (70:30, acetone:water) as solvent and a extraction time of 24 h, at 25 °C, with gentle agitation. The supernatant containing the condensed tannins was recovered by filtration through four layers of gauze and pressed. To this filtrate, the same amount of ground *Cistus ladanifer* was added as well as the solvent that allow to maintain the solid:liquid ratio of 1/5. After 24 h at 25 °C with agitation, the supernatant was filtered through four layers of gauze and pressed. The solvent was evaporated by rotary evaporation and the water was removed by freeze-drying, obtaining the condensed tannins extract (Guerreiro *et al.,* 2020). The proposed invention is distinct from this study for obtaining an extract of condensed tannins with a lower extraction time (2 h) against 48 h.

A similar process was performed by Dentinho et al. (2020), where leaves and green stems of *Cistus ladanifer* were dried and ground (1mm) and extracted with acetone:water solution (70:30, v/v) with a solid:liquid ratio of 1:10. This mixture was maintained for two days in a closed container being manually stirred at regular intervals during the day. The supernatant containing CT was then collected, filtered through 6 layers of gauze and placed on trays in a fume hood until acetone evaporation was complete. The aqueous extract was washed in a separatory funnel with petroleum ether to remove fats and pigments. The aqueous phase with CT was frozen at - 20° C and freeze-dried (Dentinho *et al.,* 2020). The present invention is distinct from this study for obtaining an extract of condensed tannins with a lower extraction time (2 h) against, at least 48 h, without use of environmentally aggressive extraction solvents, and without use of additional extraction steps.

### Summary

The present patent application discloses a method for obtaining an extract of condensed tannins obtained from aerial part of *Cistus ladanifer,* comprising the following steps:
a) drying and grinding of aerial part of *Cistus ladanifer,* with a particle size less than 1 mm (1);
b) extracting condensed tannins from the dried and ground biomass, with an ultrasonic probe (2), using 70% aqueous acetone solution at a acetone:water ratio of 70:30, with a solid/liquid ratio of 1:5 and a duration of between 10 and 60 minutes, at a temperature between 20 and 30 °C;
c) filtering the mixture coming from step (b);
d) extracting condensed tannins from a new dried biomass, using the filtered extract obtained in step (c), completing the volume with 70% aqueous acetone solution at a acetone:water ratio of 70:30, with a solid/liquid ratio of 1:5, using an ultrasonic probe (4) with a duration of between 10 and 60 minutes, at a temperature between 20 and 30 °C;
e) filtering the mixture coming from step (d);
f) concentrating the filtered extract coming from step (e) under vacuum;
g) freeze-drying the concentrate extract of step (f) under vacuum.

In one embodiment, the aerial part of *Cistus ladanifer* is any portion of the plant selected from leaves, soft stems and reproductive organs, such as flower buds, flowers and seed heads.

In one embodiment, the duration of steps (b) and (d) is 60 minutes.

In another embodiment, the temperature of steps (b) and (d) is 25 °C.

### Disclosure/Detailed Description

The present patent application discloses a new method to obtain an extract of condensed tannins from aerial part of *Cistus ladanifer,* using ultrasound probe extraction and aqueous acetone solution as extraction solvent. With the methodology described in this patent application for extraction of condensed tannins, without use toxic extraction solvents, a reduction in the extraction time are achieved as compared to the methodology of the prior art.

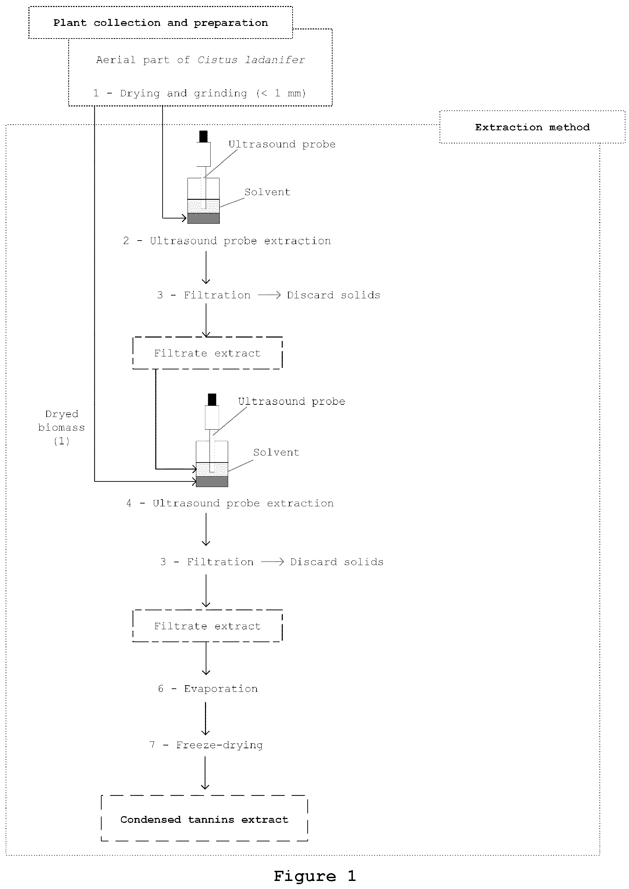

The extraction is carried out using ultrasound as extraction method and aqueous acetone solution. After extraction process, no additional extraction and/or purification steps are required (Fig. 1). The process described herein allows to obtain an extract of condensed tannins starting from the aerial part of *Cistus ladanifer* and comprised the following steps:
a) drying and grinding of aerial part of *Cistus ladanifer,* with a particle size less than 1 mm;
b) extracting condensed tannins from the dried and ground biomass, with an ultrasonic probe, using 70% aqueous acetone solution (70:30, acetone:water), with a solid/liquid ratio of 1/5 and a duration of between 10 and 60 minutes, at a temperature between 20 and 30 °C;
c) filtering the mixture coming from step (b);
d) extracting condensed tannins from a new dried biomass, using the filtered extract obtained in step (c), completing the volume with 70% aqueous acetone solution (70:30, acetone:water), with a solid/liquid ratio of 1/5, using an ultrasonic probe with a duration of between 10 and 60 minutes, at a temperature between 20 and 30 °C;
e) filtering the mixture coming from step (d);
f) concentrating the filtered extract coming from step (e) under vacuum;
g) freeze-drying the concentrate extract coming from step (f) under vacuum.
This process allows to obtain extracts of condensed tannins, by using the ultrasound extraction method with aqueous acetone solution.

### Plant collection and preparation

Aerial part of *Cistus ladanifer* can include any portion of the plant as leaves, soft stems and reproductive organs (flower buds, flowers and seed heads). Fresh aerial parts are manually harvested and it is immediately dried at room temperature, in a ventilated space, until a constant weight, indicative of complete removal of the moisture present in fresh material. After obtaining a constant weight, the material is ground using any commercial mill, with a particle size less than 1 mm (1). After grinding, the material is stored at room temperature, protected from light to avoid deterioration of its compounds of interest.

### Extraction method

The method for the extraction of condensed tannins consists of placing together the dried biomass (1) and the solvent, in a container protected from light, subjected to ultrasound probe equipment 2, at a particular biomass/solvent ratio, during a particular period of time and at a particular temperature.

In the present invention, a ultrasound probe (2) is used and a 70% aqueous acetone solution (70:30, acetone:water) as extraction solvent. In the present invention, the extraction time is comprised between 10 and 60 minutes, preferably between 50 and 60 minutes. The extraction temperature is between 20 and 30 °C, preferably between 23 and 27 °C. The biomass/solvent ratio is preferably 1:5.

The extracted mixture is filtered using any commercially available vacuum pump through a filter paper (3) (pore 5-13 µm).

The filtered extract, containing the condensed tannins, is used for a second extraction. The volume of the filtrate extract is measured and 70% aqueous acetone solution (70:30, acetone:water) is added to complete the same volume used in the first extraction. Then, this extract solution is added to the same amount of dried biomass used in the first extraction. An ultrasound probe (4) is used and the extraction time is comprised between 10 and 60 minutes, preferably between 50 and 60 minutes. The extraction temperature is between 20 and 30 °C, preferably between 23 and 27 °C. The biomass/solvent ratio is preferably 1/5.

The extracted mixture is filtered using any commercially available vacuum pump through a filter paper (5) (pore 5-13 µm). The filtered extract is concentrated by the evaporation (6) of the acetone, using any commercially available rotary evaporator under vacuum, at 30 °C. The concentrated extract is freeze-dried (7) in any commercially available freeze-dryer, under vacuum, for the removal of the water, until a constant weight of the extract.

The final extract of condensed tannins is quantified for the content of condensed tannins using a butanol-HCl method according to Porter et al. (1986), and the results are expressed as mg of Delphinidin chloride equivalents/g of lyophilizate, using Delphinidin chloride as standard.

### Example

The dried and ground biomass, in a biomass/solvent ratio of 1/5, with 70% aqueous acetone solution as solvent, was used to obtain the extract rich in condensed tannins. Ultrasonic extraction was performed using an ultrasound probe device, 20 kHz, with a continuous pulse, an amplitude of 15%, an extraction temperature of 25 °C and extraction time of 60 minutes. Then, the mixture obtained was filtered. The filtered extract, containing the condensed tannins, was added to a portion of 70% aqueous acetone solution, to complete the volume of solvent used for the first extraction. Dried and ground biomass was added to this extract mixture, in the same biomass/solvent ratio of 1:5. The extraction was repeated, using an ultrasound probe device, 20 kHz, with a continuous pulse, an amplitude of 15%, an extraction temperature of 25 °C and extraction time of 60 minutes. Then, the mixture obtained was filtered and the solvent was evaporated by rotator evaporation at 30 °C and the water was removed by freeze-drying.

The final extract presented a concentration of 76.1 mg of Delphinidin chloride equivalents/g of lyophilizate.

Several features are described hereafter that can each be used independently of one another or with any combination of the other features. However, any individual feature might not address any of the problems discussed above or might only address one of the problems discussed above. Some of the problems discussed above might not be fully addressed by any of the features described herein. Although headings are provided, information related to a particular heading, but not found in the section having that heading, may also be found elsewhere in the specification.

### Brief description of drawings

For easier understanding of this application, figures are attached in the annex which nevertheless are not intended to limit the technique disclosed herein.

Figure 1 illustrates a diagram of the extraction process according to the invention.

### Description of the embodiments

Now, preferred embodiments of the present application will be described in detail. However, they are not intended to limit the scope of this application.

The present patent application discloses a method for obtaining an extract of condensed tannins obtained from aerial part of *Cistus ladanifer,* characterized in that it comprises the following steps:
a) drying and grinding of aerial part of *Cistus ladanifer,* with a particle size less than 1 mm (1);
b) extracting condensed tannins from the dried and ground biomass, with an ultrasonic probe (2), using 70% aqueous acetone solution at a acetone:water ratio of 70:30, with a solid/liquid ratio of 1:5 and a duration of between 10 and 60 minutes, at a temperature between 20 and 30 °C;
c) filtering the mixture coming from step (b);
d) extracting condensed tannins from a new dried biomass, using the filtered extract obtained in step (c), completing the volume with 70% aqueous acetone solution at a acetone:water ratio of 70:30, with a solid/liquid ratio of 1:5, using an ultrasonic probe (4) with a duration of between 10 and 60 minutes, at a temperature between 20 and 30 °C;
e) filtering the mixture coming from step (d);
f) concentrating the filtered extract coming from step (e) under vacuum;
g) freeze-drying the concentrate extract of step (f) under vacuum.

In one embodiment, the aerial part of *Cistus ladanifer* is any portion of the plant selected from leaves, soft stems and reproductive organs, such as flower buds, flowers and seed heads.

In one embodiment, the duration of steps (b) and (d) is 60 minutes.

In another embodiment, the temperature of steps (b) and (d) is 25 °C.

This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

### References

Dentinho, M. T. P., Belo, A. T., & Bessa, R. J. B. (2014). Digestion, ruminal fermentation and microbial nitrogen supply in sheep fed soybean meal treated with Cistus ladanifer L. tannins. Small Ruminant Research, 119(1-3), 57-64.
Dentinho, M. T. P., Paulos, K., Francisco, A., Belo, A. T., Jerónimo, E., Almeida, J., et al. (2020). Effect of soybean meal treatment with Cistus ladanifer condensed tannins in growth performance, carcass and meat quality of lambs. Livestock Science, 236, 104021.
Guerreiro, O., Alves, S. P., Costa, M., Cabo, A., Duarte, M. F., Jerónimo, E., et al. (2016). Effects of extracts obtained from Cistus ladanifer L. on in vitro rumen biohydrogenation. Animal Feed Science and Technology, 219, 304-312.
Guerreiro, O., Alves, S. P., Soldado, D., Cachucho, L., Almeida, J. M., Francisco, A., et al. (2020). Inclusion of the aerial part and condensed tannin extract from Cistus ladanifer L. in lamb diets - Effects on growth performance, carcass and meat quality and fatty acid composition of intramuscular and subcutaneous fat. Meat Science, 160*.*
Hagerman, A. E. (1992). Tannin-protein interactions. In H. C. a. H. MT (Ed.), Phenolic compounds in food and their effects on Health. I. Analysis, occurrence and chemistry (pp. 236-247). Washington, DC: ACS Publications.
Jerónimo, E., Pinheiro, C., Lamy, E., Dentinho, M. T., Sales-Baptista, E., Lopes, O., et al. (2016). Tannins in ruminant nutrition: Impact on animal performance and quality of edible products. In C. A. Combs (Ed.), Tannins: Biochemistry, Food Sources and Nutritional Properties (pp. 121-168). Hauppauge, NY, USA: Nova Science Publisher Inc.
Min, B. R., Barry, T. N., Attwood, G. T., & McNabb, W. C. (2003). The effect of condensed tannins on the nutrition and health of ruminants fed fresh temperate forages: a review. Animal Feed Science and Technology, 106(1-4), 3-19.
Porter, L. J., Hrstich, L. N., & Chan, B. G. (1986). The conversion of procyanidins and prodelphinidins to cyanidin and delphinidin. Phytochemistry, 25(1), 223-230.
Vasta, V., & Luciano, G. (2011). The effects of dietary consumption of plants secondary compounds on small ruminants' products quality. Small Ruminant Research, 101(1-3), 150-159.

## Claims

1. Method for obtaining an extract of condensed tannins from aerial part of *Cistus ladanifer,* **characterized in that** it comprises the following steps:
a) drying and grinding of aerial part of *Cistus ladanifer,* with a particle size less than 1 mm;
b) extracting condensed tannins from the dried and ground biomass, with an ultrasonic probe, using 70% aqueous acetone solution at a acetone:water ratio of 70:30, a solid/liquid ratio of 1:5 and a duration of between 10 and 60 minutes, at a temperature between 20 and 30 °C;
c) filtering the mixture coming from step (b);
d) extracting condensed tannins from a new dried biomass, using the filtered extract obtained in step (c), completing the volume with 70% aqueous acetone solution at a acetone:water ratio of 70:30, a solid/liquid ratio of 1:5, using an ultrasonic probe with a duration of between 10 and 60 minutes, at a temperature between 20 and 30 °C;
e) filtering the mixture coming from step (d);
f) concentrating the filtered extract coming from step (e) under vacuum;
g) freeze-drying the concentrate extract of step (f) under vacuum.

2. The method for obtaining an extract of condensed tannins from aerial part of *Cistus ladanifer* according to claim 1, wherein the aerial part of *Cistus ladanifer* is any portion of the plant selected from leaves, soft stems and reproductive organs, such as flower buds, flowers and seed heads.

3. The method for obtaining an extract of condensed tannins from aerial part of *Cistus ladanifer* according to any of claims 1-2, wherein the duration of steps (b) and (d) is 60 minutes.

4. The method for obtaining an extract of condensed tannins from aerial part of *Cistus ladanifer* according to any of claims 1-3, wherein the temperature of steps (b) and (d) is 25 °C.
